# EUROPEAN PATENT SPECIFICATION

(11) **EP 3 250 929 B1**
(45) Date of publication and mention of the grant of the patent: **25.03.2020**
(21) Application number: 16702782.0
(22) Date of filing: 29.01.2016
(51) Int. Cl.: G01N 33/574, C07K 16/00, G01N 33/68

(54) **DIAGNOSIS OF RISK OF UROTHELIAL CANCER**
DIAGNOSE DES RISIKOS AUF UROTHELKREBS
DIAGNOSTIC DE RISQUE DE CANCER UROTHÉLIAL

(30) Priority: 30.01.2015 GB 201501597
(43) Date of publication of application: 06.12.2017
(73) Proprietor: Randox Laboratories Ltd., Northern Ireland (GB)
(72) Inventor: RUDDOCK, Mark W., Co Antrim Northern Ireland BT29 4QY (IE); REID, Cherith N., Co Antrim Northern Ireland BT29 4QY (IE); WILLIAMSON, Kate E., Belfast County Antrim Northern Ireland BT9 7BL (IE); LAMONT, John, Co Antrim Northern Ireland BT29 4QY (IE); FITZGERALD, Peter, Co Antrim Northern Ireland BT29 4QY (IE)
(74) Representative: Gill Jennings & Every LLP
(86) International application number: PCT/GB2016/050200
(87) International publication number: WO 2016/120633

(56) References cited:
- WO-A1-2011/012901
- WO-A1-2011/035097
- WO-A1-2012/128108
- WO-A1-2015/150834
- R L ATTANOOS ET AL: "'Pseudomesotheliomatous' carcinomas of the pleura: a 10-year analysis of cases from the Environmental Lung Disease Research Group, Cardiff", HISTOPATHOLOGY., vol. 43, no. 5, 1 November 2003 (2003-11-01), pages 444-452, XP055254851, GB ISSN: 0309-0167, DOI: 10.1046/j.1365-2559.2003.01674.x
- CHUN-TE WU ET AL: "Thrombomodulin expression regulates tumorigenesis in bladder cancer", BMC CANCER, BIOMED CENTRAL, LONDON, GB, vol. 14, no. 1, 28 May 2014 (2014-05-28), page 375, XP021187567, ISSN: 1471-2407, DOI: 10.1186/1471-2407-14-375
- GILMOUR P S ET AL: "Cardiovascular and blood coagulative effects of pulmonary zinc exposure", TOXICOLOGY AND APPLIED PHARMACOLOGY, ACADEMIC PRESS, AMSTERDAM, NL, vol. 211, no. 1, 15 February 2006 (2006-02-15), pages 41-52, XP024895998, ISSN: 0041-008X, DOI: 10.1016/J.TAAP.2005.06.002 [retrieved on 2006-02-15]
- EDANO T ET AL: "IMPORTANCE OF SIALIC ACID IN RECOMBINANT THROMBOMODULIN IN TERMS OF PHARMACOKINETICS AND SEPARATION OF DESIALYZED GLYCOPROTEIN", BIOLOGICAL & PHARMACEUTICAL BULLETIN (OF JA, PHARMACEUTICAL SOCIETY OF JAPAN, TOKYO, JP, vol. 21, no. 4, 1 April 1998 (1998-04-01), pages 382-385, XP009034122, ISSN: 0918-6158

## Description

### Field of Invention

The invention relates to a method of detecting an increased risk of cancer, and in particular urothelial cancer (UC), in subjects with a history of exposure to chemicals.

### Background of the Invention

UC is a leading cause of death worldwide.

A link between occupational exposure to chemicals and UC was reported by Ludwig Rehn in 1895 following an observational study of German dye workers [1]. Since then the list of chemical agents linked to UC has grown. Currently this list includes 1,1-dichloroethane, β-naphthylamine or 2-naphthylamine, 4,4'-methylenebis (2-chloroaniline) MOCA, 4-aminobiphenyl, aromatic amines, arsenic, auramine, benzidine, xenylamine, benzidine-derived dyes, aniline and azo dyes, benzo(a)pyrene, chlordimeform/4-COT, coal tars, direct black 38, direct blue 6, direct brown 95, nitrobiphenyl, o-toluidine, polycyclic aromatic hydrocarbons, tobacco smoke, carbaryl, chlorination by-products, chlornaphazine, chlorophenols, creosotes, methylenedianiline, propoxur, solvent, trihalomethanes, antimony, asbestos, bifenthrin, cacodylic acid, carbamates, chromium, leather dust, nickel, cadmium, dichloropropene, diesel exhaust, dioxins/ 2,3,7,8-tetrachlorodibenzo-p-dioxin, lead, nitrosamines, o-phenyl-phenol, organochlorine pesticides, p-cresidine, pesticides, pyrethins/pyrethroids, saccharin, tetrachloroethylene (PCE), phenyl β-naphthylamine, 2-mercaptobenzothiazole, solvent red 164 as dye penetrant, solvent red 19 as dye penetrant or anti-rust agent, dinitrotoluenes, diazo printing ink, derivatives or combustion products of fossil fuels, anti-neoplastic drugs such as cyclophosphamide, ifosfamide and cisplatin, diabetic medications such as pioglitazone, metformin and glimepiride, and chronic use of pain killers such as paracetamol, phenacetin and ibuprofen. While health and safety regulations may limit occupational exposure to harmful chemicals, related cases of UC remain high as the latency for cancer development after exposure may be as long as 50 years. Therefore, new biomarkers and methods of identifying subjects at higher risk of bladder cancer as a consequence of chemical exposure are required.

UC is the most common malignancy detected in patients who present with haematuria and is the fourth most common cancer in men. UC was the estimated cause of death in 150,200 people, worldwide in 2008 [2]. Diagnosis and urgently needed treatment of aggressive UC can be delayed when triage is ineffective [3]. Therefore, new biomarkers and methods of identifying patients at highest risk of UC remains an unmet clinical need.

The incidence of UC increases with age and is associated with many risk factors, for example smoking. Smoking increases the risk of UC up to 4-fold, and extended duration or amount of smoking leads to increased risk while stopping smoking is associated with a decreased risk [4]. Low fluid consumption has also been suggested to increase the risk of UC by allowing harmful chemicals to concentrate in urine and recent studies have shown that higher fluid intake may lower a person's risk [5]. At the time of diagnosis, approximately 70% of patients diagnosed with UC have tumours that are pathologically staged as pTa, pT1 or carcinoma *in situ* (CIS) i.e., non-muscle invasive (NMI) disease and these patients generally have a good prognosis. When a patient's tumour is pathologically defined as ≥ T1G3 UC, the patient is deemed to have a higher risk of progression to a more life threatening disease. Muscle invasive (MI) UC encompasses all pathological stages ≥ pT2. The risk parameters that are currently used to tailor follow-up for patients diagnosed with UC, include pathological parameters i.e., grade, stage and associated CIS, together with resistance to Bacille Calmette-Guerin (BCG) treatment. However, it is not always possible to correctly predict the outcome for patients. This is largely attributable to the molecular heterogeneity within tumours which means that a spectrum of outcomes, spanning from negligible risk to life threatening prognosis, exists within the same pathologically classified groups. For this reason, all patients with NMI disease have frequent surveillance cystoscopies and those with MI have radiological surveillance for lymph node recurrence or distant metastasis.

The ability to identify patients who are at risk of aggressive bladder cancer attributable to past chemical exposure using a biomarker assay could enable clinicians to improve risk stratification decisions. This could result in improved patient outcomes at reduced costs.

The publication WO 2011/035097 discloses methods and compositions for monitoring, diagnosis, prognosis and determination of treatment in regimens in subjects suffering from or suspected of having a renal injury. The publication WO 2011/012901 discloses a method for the detection of, or the risk of, bladder cancer in a patient, comprising the step of detecting the presence of a combination of biomarkers. The publication WO 2012/128108 addresses the problem of providing an objective and simple means for evaluating the history of exposure to asbestos. Attanoos and Gibbs (2003) relates to a clinicopathological study of diffuse serosal neoplasms of epithelial histogenesis which clinically and pathologically mimic malignant pleural mesothelioma. Wu et al. (2014) discloses that thrombomodulin expression regulates tumorigenesis in bladder cancer. Gilmour et al. (2006) relates to the cardiovascular and blood coagulative effects of pulmonary zinc exposure. Edano et al. (1998) discloses the importance of sialic acid in recombinant thrombomodulin in terms of pharmacokinetics and separation of desialyzed glycoprotein.

### Summary of the Invention

The present invention describes a biomarker that can be measured in urine and used to identify individuals exposed to chemical(s) who have an increased risk of developing bladder cancer as defined in the claims. The biomarker can be measured in a sample obtained from a subject.

According to a first aspect, the present invention provides a method for detecting individuals with a history of exposure to chemical(s) comprising measuring the concentration of thrombomodulin in a sample isolated from a subject, and determining whether the concentration of thrombomodulin is increased compared to control reference levels, wherein the subject has had or is suspected of having prior history of exposure to a chemical selected from the list comprising 1,1-dichloroethane, 2-mercaptobenzothiazole, β-naphthylamine or 2-naphthylamine, 4,4'-Methylenebis(2-chloroaniline) MOCA, 4-aminobiphenyl, aromatic amines, arsenic, auramine, benzidine, xenylamine, benzidine-derived dyes, aniline and azo dyes, benzo(a)pyrene, chlordimeform/4-COT, direct black 38, direct blue 6, direct brown 95, nitrobiphenyl, o-toluidine, polycyclic aromatic hydrocarbons, carbaryl, chlornaphazine, chlorophenols, creosotes, methylenedianiline, propoxur, trihalomethanes, antimony, asbestos, bifenthrin, cacodylic acid, carbamates, chromium, cadmium, nickel, leather dust, dichloropropene, nitrosamines, o-phenyl-phenol, organochlorine pesticides, p-cresidine, pesticides, pyrethins/pyrethroids, saccharin, tetrachloroethylene, phenyl β-naphthylamine, solvent red 164 as dye penetrant, solvent red 19 as dye penetrant or anti-rust agent, dinitrotoluenes, diazo printing ink, anti-neoplastic drugs, diabetic medications and pain killers.

According to a second aspect, the present invention provides a method for detecting individuals with a history of exposure to chemical(s) who have an increased risk of developing cancer, comprising measuring the concentration of urinary thrombomodulin in a sample isolated from the subject, and determining whether the concentration of thrombomodulin is increased compared to a control, wherein the subject has had or is suspected of having prior exposure to a chemical(s) selected from the list comprising 1,1-dichloroethane, 2-mercaptobenzothiazole, β-naphthylamine or 2-naphthylamine, 4,4'-Methylenebis(2-chloroaniline) MOCA, 4-aminobiphenyl, aromatic amines, arsenic, auramine, benzidine, xenylamine, benzidine-derived dyes, aniline and azo dyes, benzo(a)pyrene, chlordimeform/4-COT, direct black 38, direct blue 6, direct brown 95, nitrobiphenyl, o-toluidine, polycyclic aromatic hydrocarbons, carbaryl, chlornaphazine, chlorophenols, creosotes, methylenedianiline, propoxur, trihalomethanes, antimony, asbestos, bifenthrin, cacodylic acid, carbamates, chromium, cadmium, nickel, leather dust, dichloropropene, , nitrosamines, o-phenyl-phenol, organochlorine pesticides, p-cresidine, pesticides, pyrethins/pyrethroids, saccharin, tetrachloroethylene, phenyl β-naphthylamine, solvent red 164 as dye penetrant, solvent red 19 as dye penetrant, or anti-rust agent, dinitrotoluenes, diazo printing ink, anti-neoplastic drugs, diabetic medications and pain killers, wherein the cancer is bladder cancer.

### Description of the Drawings

Figure 1 shows a tumour biopsy sample from a patient with a history of chemical exposure which illustrates surface micropapillary features and small clusters of tumour cells that lack a fibrovascular core (x40 magnification).
Figure 2 shows Venn charts comparing the relationships between history of exposure to chemicals and smoking.

### Description of the Invention

The present invention is based on the finding that the level of thrombomodulin in urine and/or blood samples isolated from a subject are altered in a subject who has been exposed to chemicals compared to a subject who has not been so exposed, i.e. a 'control'. The identification of such subjects with elevated levels of thrombomodulin and therefore at higher risk of bladder cancer will act as a valuable adjunct to risk stratification. This is advantageous since it enables early bladder cancer diagnosis and a more favourable outcome for the individual.

The present invention provides a method for detecting increased risk for UC in subjects with a history of exposure to a chemical(s) by measuring the level of thrombomodulin in a sample from a subject and comparing the measured level with a "control" level i.e. that measured in subjects without a history of exposure to chemicals as defined in the claims. The method of detection and monitoring of exposure of individuals to chemicals, and the information it provides is useful as it can inform medical, environmental, occupational, professional and lifestyle decisions.

A number of biomarkers present in a sample isolated from a patient suspected of a history of exposure to a chemical(s) or having an increased risk of bladder cancer may have levels which are different to those of a "control". However, the levels of some of the biomarkers that are different compared to a "control" may not show a strong enough correlation with exposure to a chemical(s) or increased risk of bladder cancer such that they may be used to indicate exposure to a chemical(s) or an increased risk of bladder cancer with an acceptable accuracy.

The increased risk of bladder cancer identified using the invention may last for a prolonged period of time after testing. For example, the effects of exposure to chemicals are known to be long lasting, and the latency period for subsequent development of tumours can be as long as 50 years, dependent on the specific chemical exposure. Accordingly, the methods of the present invention provide for identification of long term risks for the test subject wherein a tumour may develop within up to 50 years. The methods of the invention could be used to monitor a subject's risk of developing cancer and may allow determination of any changes in risk levels over time. Where a subject seeks to adjust their risk of developing cancer, it is possible using the methods of the invention to determine if this adjustment is successful and to monitor this adjustment. Where a subject is treated to reduce their risk of developing cancer, the methods of the present invention may be used to monitor the progress of treatment and to measure reduction in the risk of developing cancer.

The individual biomarker identified by the present inventors as having an increased level in subjects exposed to a chemical(s) or having increased risk of bladder cancer compared to a 'control' value is thrombomodulin.

The term "bladder cancer" is understood to include urothelial carcinoma (UC), bladder squamous cell carcinoma or bladder adenocarcinoma. Preferably, the bladder cancer is urothelial carcinoma.

Grading of a cancer provides an indication to the physician of how the cancer is likely to develop and if it is likely to spread to other sites in the subject's body. Various systems are used to grade different types of cancers. In the case of bladder cancer "grade 1" indicates that cancer cells are slow growing and similar to normal cells (well differentiated) with a low likelihood of spreading. "Grade 2" bladder cancer indicates that the cancer cells look slightly abnormal and are growing more quickly than normal differentiated cells, and "grade 3" indicates that the cancer cells are growing rapidly, are highly abnormal in appearance (poorly differentiated) and are very likely to spread. Low grade bladder cancer corresponds to grades I and II, while high grade cancers are grade III.

Staging of cancer indicates the depth of invasion into the urothelium by the cancer and whether it has already spread to other sites. This information is useful to the physician in deciding on how to treat the individual cancer sufferer. Stage pTa indicates that the cancer is small and limited to the urothelium or bladder lining. These superficial pTa tumors usually have a good prognosis. Although Carcinoma *in situ* (CIS) indicates that the tumor is restricted to the inside layer of the urothelium, the presence of CIS in a tumor is associated with increased risk of progression. Stage pT1 indicates that the cancer has grown into the connective tissue layer underneath the urothelium, while Stage 2 cancer has grown through the connective tissue layer and entered the muscle tissue of the bladder wall. Stage 3 cancer has entered the fat tissue outside the bladder and may spread to nearby organs including the prostate or uterus. Stage 4 bladder cancer has spread to the wall of the abdomen or pelvis, to the lymph nodes and perhaps to other parts of the body. When bladder cancer spreads to other organs, it most frequently goes to the lungs, liver or bones.

"Non-muscle invasive" bladder cancer is also known as 'early' or 'superficial' cancer, and it refers to stage pTa, CIS and pT1tumours. "Invasive" bladder cancer refers to stage 2 and stage 3 cancers. "Advanced" bladder cancer refers to stage 4 cancer.

The methods of the present invention allow identification of those subjects at increased risk of having aggressive variants or of developing more advanced cancer. Subjects suffering from bladder cancer who were identified as having a history of exposure to a chemical showed a higher likelihood of having more advanced cancer compared to those subjects who were not so exposed. Accordingly, measurement of thrombomodulin levels in urine of subjects suffering from bladder cancer allows identification of those at highest risk of having advanced bladder cancer. Such subjects may be prioritised for increased frequency of surveillance. The terms "subject" and "patient" are used interchangeably herein and refer to a mammal including a non-primate (e.g. a cow, pig, horse, dog, cat, rat and mouse) and a primate (e.g. a monkey and human). Preferably the subject or patient is a human.

The subject may be non-symptomatic, and the invention may therefore be used to screen individuals who work in high risk occupations without knowing that have been exposed, or have a history of exposure to chemicals.

Frequently however, the subject is a patient presenting with haematuria. For the avoidance of doubt, the term "haematuria" refers to the presence of red blood cells and/or haemoglobin in the urine. Haematuria may be caused by a number of conditions, such as bladder cancer, benign prostate enlargement, kidney stones and infection, prostate cancer, renal cell carcinoma or urinary tract infections. In a clinical setting, the clinician may make an assessment of the patient's medical history, note the patient's occupational history, age and whether they smoke and, if so, for how long. Bladder cancer most commonly occurs in the 6^{th}, 7^{th} and 8^{th} decades of life. The methods of the present invention provide means of identifying a risk of developing bladder cancer earlier in life. Subjects who have been exposed to a chemical(s) show an earlier onset of this disease. The methods of the present invention are therefore particularly useful in identifying a risk of developing bladder cancer before the age of 70 or before the age of 65. More valuably, the present invention allows identification of subjects at risk of developing bladder cancer before the age of 60 or even younger.

The term "level" refers to the measured amount, level or concentration of an analyte within a sample.

Preferably, the biomarkers are detected in a sample obtained from the subject selected from a urine sample, blood sample, i.e. serum sample or plasma sample. Preferably the sample is a urine sample.

The term "altered" refers to a changed amount or level of any measured substance, and it can mean either an increased or a decreased amount or level. Preferably the amount or level is increased.

The term "control" refers to a level of an analyte to which a corresponding level measured in a sample from a subject can be compared. The control can be a predetermined reference level that is set as a standard for comparison to individual samples, a level that is measured in a sample from a subject not exposed to chemicals or not having in increased risk of cancer due to suspected exposure to chemicals or a level measured in a sample from the same subject. For example, the control level of an analyte may be determined by analysis of a sample isolated from a person with haematuria but who does not have bladder cancer or may be the level of the biomarker understood by the skilled person to be typical for such a person. The control value of a biomarker may be determined by methods known in the art and normal values for a biomarker may be referenced from the literature from the manufacturer of an assay used to determine the biomarker level.

The term "baseline sample" refers to an earlier sample from a subject or a population of subjects that may be used or provide a measurement value that may be used as a reference for comparison with a later sample from a subject or population of subjects.

The terms "serious disease" and "serious underlying pathology" are used interchangeably herein, and refer to life-threatening conditions such as aggressive bladder cancer or other aggressive cancers.

The term "history of smoking" refers herein to whether or not the subject smokes, or has a history of having smoked. The term "smoking" is defined by the use of tobacco in any form, including cigarettes, cigars and pipe tobacco. An individual subject can be classified as positive (i.e. has a history of smoking) or negative (i.e. no history of smoking) for smoking habits.

The methods of the present invention allow identification of those subjects at highest risk of developing cancer. Subjects with a history of smoking who were identified as having suffered exposure to a chemical(s) showed a higher likelihood of having bladder cancer compared to those subjects who smoked but were not so exposed. Accordingly, measurement of thrombomodulin levels in urine of subjects with a history of smoking according to the present disclosure allows identification of those at highest risk of having bladder cancer.

"Hazardous chemicals" are those linked to an increased risk or incidence of bladder cancer, and they include chemicals strongly linked to bladder cancer, chemicals linked to bladder cancer and medications linked to bladder cancer. Chemicals strongly linked to bladder cancer are selected from the list comprising: (strong evidence) 1,1-dichloroethane, β-naphthylamine or 2-naphthylamine, 4,4'-methylenebis (2-chloroaniline) MOCA, 4-aminobiphenyl, aromatic amines, arsenic, auramine, benzidine, xenylamine, benzidine-derived dyes, aniline and azo dyes, benzo(a)pyrene, chlordimeform/4-COT, direct black 38, direct blue 6, direct brown 95, nitrobiphenyl, o-toluidine, polycyclic aromatic hydrocarbons, tobacco smoke, (good evidence) carbaryl, , chlornaphazine, chlorophenols, creosotes, methylenedianiline, propoxur, and trihalomethanes. Chemicals linked to bladder cancer (limited evidence) are selected from the list comprising: antimony, asbestos, bifenthrin, cacodylic acid, carbamates, chromium, nickel, leather dust, cadmium, dichloropropene, , nitrosamines, o-phenyl-phenol, organochlorine pesticides, p-cresidine, pesticides, pyrethins/pyrethroids, saccharin, tetrachloroethylene (PCE), phenyl β-naphthylamine, 2-mercaptobenzothiazole, solvent red 164 as dye penetrant, solvent red 19 as dye penetrant or anti-rust agent, dinitrotoluenes and diazo printing ink. Chemicals that are medications linked to bladder cancer are selected from the list comprising: anti-neoplastic drugs such as cyclophosphamide, cisplatin and ifosfamide, diabetic medications such as pioglitazone, metformin and glimepiride, and pain killers (chronic use) such as paracetamol, phenacetin and ibuprofen.

It will be understood that subjects engaged in particular occupations or activities may be at increased risk of exposure to chemicals. Such individuals with increased risk of exposure to chemicals include: sales workers in highly exposed sales jobs, e.g., hardware and fuel sales; health workers exposed to hazardous chemicals that are medications e.g. anti-neoplastic drugs; fire fighters who experience diverse chemical exposures; farmworkers who experience exposure to pesticides and herbicides; food preparers who experience exposure to heterocyclic amines produced during cooking, and by-products of the use of chlorinated sterilising agents; iron and steel workers who experience exposure to polycyclic aromatic hydrocarbons , mineral oil, etc.; miners who experience exposure to mineral oil from cutting and hydraulic systems, diesel fumes, and explosives; clerical workers who experience exposure to printing ink, mineral oil, pigments and dyes; assemblers who experience exposure to mineral oil and solder containing lead and cadmium; electronics workers who experience exposure to solder containing lead and cadmium; gas supply workers who experience exposure to polycyclic aromatic hydrocarbons, aromatic amines and brazing solder; construction workers who experience exposure to diesel, asphalt fumes, polycyclic aromatic hydrocarbons , and coal tar pitch products; workers in paper manufacture
who experience exposure to chlorination by-products; machine setters and operators; mechanics; metal casters; leather workers; printers (carbon black); workers using adhesives; and workers in tobacco product manufacture.

The determination of the level of the biomarker in the sample may be determined by commercially available methods such as an ELISA-based assay, chemical or enzymatic protein determination. Preferably, the methods of the present invention use a solid state device for determining the level of the biomarkers in the sample isolated from the patient. The solid state device comprises a substrate having an activated surface on to which an antibody to the biomarker of interest is immobilised at discreet areas of the activated surface. Preferably, the solid state device may perform multi-analyte assays such that the level of a biomarker of interest in a sample isolated from the patient may be determined simultaneously with the level of a further biomarker of interest in the sample. In this embodiment, the solid state device has a multiplicity of discrete reaction sites each bearing a desired antibody covalently bound to the substrate, and in which the surface of the substrate between the reaction sites is inert with respect to the target biomarker. The solid state, multi-analyte device may therefore exhibit little or no non-specific binding.

The device that may be used in the invention may be prepared by activating the surface of a suitable substrate, and applying an array of antibodies on to discrete sites on the surface. If desired, the other active areas may be blocked. The ligands may be bound to the substrate via a linker. In particular, it is preferred that the activated surface is reacted successively with an organosilane, a bifunctional linker and the antibody. The solid state device used in the methods of the present invention may be manufactured according to the method disclosed in, for example, GB-A-2324866. Preferably, the solid state device used in the methods of the present invention is the Biochip Array Technology system (BAT) (available from Randox Laboratories Limited). More preferably, the Evidence Evolution and Evidence Investigator apparatus (available from Randox Laboratories) may be used to determine the levels of biomarkers in the sample.

The solid state device may be used, either alone or in combination with other clinical indicators, to assess the risk of a subject having bladder cancer and/or stratify the level of risk of serious disease, wherein combination of antibodies present on the solid state device are selected according to a sub-population group that is appropriate to the subject.

At least one, but optionally two or more different solid state devices may be used for each individual subject in order to assess their risk of having bladder cancer. If multiple devices are used, each will comprise a combination of antibodies selected according to sub-population groups that are appropriate to the subject.

### Example

### Patient database

The patient database comprised clinical, demographic and biomarker levels (n = 29) measured in urine, serum and plasma collected from 156 patients with haematuria recruited to a case-control study, conducted according to Standards for the Reporting of Diagnostic accuracy studies (STARD) guidelines. Biomarker analyses in urine, serum and plasma were carried out as described previously [6].

The occupational history, exactly as it was recorded for each patient, was reviewed by three authors (MR, CR and KW). Each patient's occupational history was reviewed and classified as low risk (score = 1), moderate risk (score = 2), or high risk (score = 3) as reported previously [6]. The occupational risk score (ORS) categories were then averaged. Occupations, which were deemed as high-risk for developing bladder cancer included categories of painters [7], mechanics [8], metal, textile and electrical workers, miners, transport operators, excavating-machine operators, concierges and janitors [9-11], foundry workers [12] and hair dressers [13]. Occupations thus deemed as low risk for the development of bladder cancer included teachers, receptionists, shop assistants, accountants, civil servants and office clerks. Twenty-seven of the 156 patients (17%) were classified as having an occupational risk by two or more of the three independent reviewers were assigned as chemical exposure (CE) (Table 1); 14/27 (52%) had UC: pTa UC (n = 5), pT1 UC (n = 6), pT3a (n = 1), pT3b (n = 1) and pT4a (n = 1); 13/27 (48%) were controls (CTLs). A consultant pathologist undertook a diagnostic review of a random sample of tumor biopsies from 20 UC patients recruited to the case control study (Table 2) to ascertain the relationship between features associated with aggressive bladder cancer and each patient's history of chemical exposure.

Biomarker levels were measured on anonymised samples. Creatinine levels (µmol/L) were measured using a Daytona RX Series Clinical Analyser (Randox). Osmolality (mOsm) was measured using a Loser Micro-Osmometer (Type 15) (Loser Messtechnik, Germany). Total protein levels (mg/ml) in urine were determined by Bradford assay A₅₉₅nm (Hitachi U2800 spectrophotometer) using bovine serum albumin (BSA) as standard. Thrombomodulin, CRP, IL-4, and IL-2 were measured in urine; MCP-1 and MMP9NGAL were measured in plasma; using biochip array technology (BAT) and ELISA, as previously reported (6). All biomarker measurements were completed in triplicate (n=3).

### Statistical analyses

Fisher's exact tests were used to explore the associations between CE, diagnosis and tumour stage and grade; ANOVA, prior to t-test, to investigate age across UC and CTL with/without CE; and Zelen's exact test [14] using the NSM3 R package [15] to explore the three way association across final diagnosis (UC vs CTL), chemical exposure (CE vs NCE) and smoking (non-smoker vs smoker).

### Associations between biomarker levels and Chemical Exposure

Biomarkers with non-zero coefficients were pre-selected using Lasso in conjunction with the glmnet function in the R package [16,17], repeating each bootstrap analysis 100 times. The minimal λ (0.03636) was chosen by a 10 fold cross-validation procedure. Levels of the pre-selected biomarkers were log₁₀ transformed prior to analyses for differential levels across the CE and NCE subpopulations using Welch's t-test and applying a false detection rate (FDR) method. Independence of the CE-associated increased biomarker levels were confirmed by analysing the UC and CTL subpopulations separately using one-sided t-tests. Differential expression of thrombomodulin levels across medication classes and across the clinical subpopulations were analysed using one sided t-tests.

**Table 1 Occupational histories of patients deemed at risk of bladder cancer**

| **Diagnosis** | **Present Occupation** | **Past occupation** |
|---|---|---|
| cancer | TIMBER YARD-PAINTER/SPRAYER | SUPERVISOR B&Q |
| control | RETIRED | ROAD SERVICE MAINTAINANCE |
| cancer | RETIRED | CAR PAINTER |
| control | RETIRED | BUILDING (SKILLED LABOURER) |
| control | RETIRED | FOREMAN-WORKS MINISTRY |
| control | WINDOW BLIND FITTER | MIXED DYE FOR FABRIC FACTORY |
| cancer | RETIRED | MARINE ENGINEER |
| control | RETIRED | NEWSPAPER PRODUCTION PHOTOGRAPHER |
| cancer | RETIRED | LAND SCAPE CONTRACTOR |
| control | RECREATION PARK ATTENDANT | COAL MAN |
| cancer | RETIRED | LORRY DRIVER, AGRICULTURAL SPRAYER |
| control | TAXI DRIVER | MOULDING FACTORY |
| cancer | ENGINEERING-MECHANICAL | ENGINEER |
| control | RETIRED | CARPET CLEANER |
| cancer | RETIRED | PROCESS OPERATOR (CAR MANUFACTURE) |
| control | CLERGY MAN | TV TECHNICIAN, RAF RADIO INSPECTOR |
| cancer | RETIRED | FIREMAN |
| cancer | PRODUCTION WORKER | |
| cancer | RETIRED | LORRY DRIVER |
| cancer | RETIRED | SERVICE ENGINEER |
| cancer | RETIRED | MECHANICAL ENGINEER |
| cancer | UNEMPLOYED | CABLE JOINER |
| control | JOINER | |
| cancer | RETIRED | SCHOOL CARE TAKER, TYRE MAKING |
| control | WAREHOUSE, PLASTIC MANUFACTURER | NEWSPAPER PRODUCTION PHOTOGRAPHER |
| control | LORRY DRIVER | CAR MECHANIC, CAR VALETING |
| control | RETIRED | LABOURER |

**Table 2 Pathological review of 20 UC patients**

| **Age** | **Smoking Yrs** | **Chemical contact** | **Grade** | **Stage** | **Inflammation** | **Pattern** | **CIS** | **LVI** |
|---|---|---|---|---|---|---|---|---|
| Urothelial cancers from patients (n = 8) with no chemical exposure | | | | | | | | |
| <60 | 0 | NCE | 3 | T1 | 1 | nested | present | no |
| >60 | >10 | NCE | CIS | TIS | 1 | CIS | present | no |
| >60 | 0 | NCE | 3 | T2 | 1 | papillary | absent | no |
| >60 | 0 | NCE | 3 | T1 | 1 | papillary | present | no |
| >60 | >10 | NCE | 3 | TA | 2 | papillary | absent | no |
| >60 | >10 | NCE | 2 | TA | 0 | papillary | absent | no |
| >60 | >10 | NCE | 3 | T1 | 2 | papillary | present | no |
| >60 | >10 | NCE | 3 | T3A | 1 | nested | present | yes |

| Urothelial cancers from patients with a history of chemical exposure (CE) | | | | | | | | |
|---|---|---|---|---|---|---|---|---|
| >60* | <10 | CE | 2# | TA | 3 | sessile | absent | no |
| | | | 2 | TA | 0 | papillary | absent | no |
| | | | 3 | TA | 0 | papillary | present | no |
| >60* | >10 | CE | 2 | TA | 0 | papillary | absent | no |
| | | | 2 | TA | 2 | papillary | absent | no |
| >60* | >10 | CE | 1 # | TA | 0 | papillary | absent | no |
| | | | 2 | TA | 0 | papillary | absent | no |
| >60 | >10 | CE | 2 # | TA | 0 | papillary | absent | no |
| | | | 3 | T1 | 2 | papillary | present | yes |
| >60 | >10 | CE | 3 | T1 | 1 | micro-papillary | present | yes |
| <60 | >10 | CE | 3 | T4A | 1 | nested | absent | yes |
| >60 | >10 | CE | 3 | T2 | 1 | micro-papillary | present | yes |
| | | | 3 | T2 | 3 | micro-papillary & nested | present | yes |
| <60 | >10 | CE | 0 | | 0 | papillary | absent | no |
| | | | 3 | TA | 0 | papillary | absent | no |
| <60 | >10 | CE | 0 # | TA | 0 | papillary | absent | no |
| | | | 1 | TA | 1 | papillary | absent | no |
| | | | 3 | TA | 3 | papillary | absent | no |
| >60 | 0 | CE | 3 | T2 | 1 | nested | absent | no |
| | | | 3 | T2 | 1 | squamous diff | absent | no |
| <60 | >10 | CE | 3 | TA | 1 | micro-papillary | absent | no |
| <60 | >10 | CE | 2 | TA | 0 | papillary | absent | no |

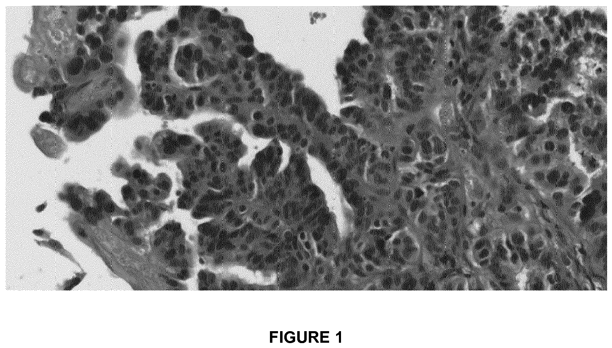

Three out of 12 (25%) patients with a history of chemical exposure (CE) exhibited features of micropapillary variant [18] UC (Figure 1); 8/12 (66.6%) CE patients had recurrences, four of which were higher grade/stage disease #. No evidence of micropapillary features and no recurrences were recorded for the eight patients with no history of CE. Inflammation: 0 absent, 1 = +, 2 = ++, 3 = +++; LVI lymphovascular invasion. *patients with incomplete biomarker data who were not included in the biomarker analyse

**Table 3 Characteristics of patients**

| | **CTL** | | **UC** | | **Total (% cohort)** |
|---|---|---|---|---|---|
| | **NCE** | **CE** | **NCE** | **CE** | |
| | **63** | **13** | **66** | **14** | **156 (100.0)** |

| Age: | | | | | |
|---|---|---|---|---|---|
| Age (mean (SD)) | 54.3 (19.1) | 54.1 (16.7) | 70.2 (8.1) | 64.1 (8.4)* | 156 (100.0) |
| | | | | | |

| Final diagnosis: | (% column) | (% column) | (% column) | (% column) | |
|---|---|---|---|---|---|
| No diagnosis | 27 (42.9) | 9 (69.2) | 0 | 0 | 36 (23.1) |
| Benign pathologies | 5 (7.9) | 1 (7.7) | 0 | 0 | 6 (3.85) |
| Stones and inflammation | 15 (23.8) | 1 (7.7) | 0 | 0 | 16 (10.3) |
| BPE | 10 (15.9) | 2 (15.4) | 0 | 0 | 12 (7.7) |
| Other cancers | 6 (9.5) | 0 | 0 | 0 | 6 (3.85) |
| NMI UC | N/A | N/A | 51 (77.3) | 11 (78.6) | 62 (39.7) |
| MI UC | N/A | N/A | 15 (22.7) | 3 (21.4) | 18 (11.5) |
| | | | | | |
| Grade 1/2 UC | N/A | N/A | 40 (62.5) | 3 (21.4) | 43 (53.8) |
| Grade 3 UC | N/A | N/A | 24 (37.5) | 11 (78.5) | 35 (43.8) |
| | | | | | |

| Dipstick blood: | | | | | |
|---|---|---|---|---|---|
| | | | | | |
| -ve | 23 (36.5) | 5 (38.5) | 12 (18.2) | 3 (21.4) | 43 (25.6) |
| + | 13 (20.6) | 5 (38.5) | 24 (36.4) | 2 (14.3) | 44 (28.2) |
| ++ | 9 (14.3) | 0 | 4 (6.0) | 1 (7.1) | 14 (9.0) |
| +++ | 17 (27.0) | 3 (23.0) | 22 (33.3) | 5 (35.7) | 47 (30.1) |
| ++++ | 1 (1.6) | 0 | 4 (6.0) | 3 (21.4) | 8 (5.0) |
| | | | | | |

| Smoking: | | | | | |
|---|---|---|---|---|---|
| Smokers | 37 (58.7) | 4 (30.8) | 47 (71.2) | 13 (92.9) | 101 (64.7) |
| Smoking years (mean (SD)) | 26.0 (16.2) | 21.3 (8.5) | 35.4 (14.5) | 37.4 (9.6) | N/A |

Urothelial cancer (UC) patients with a history of chemical exposure (CE) were significantly younger than UC patients with no chemical exposure (NCE). Two UC NCE patients had tumours that were ungraded (data for 64/66 UC NCE patients are presented). BPE benign prostate enlargement, N/A not available, NMI non- muscle invasive, MI muscle invasive, CTL control.

### Association between tumour grade and chemical exposure

When grade 1 (n = 4) and grade 2 tumours (n = 39) were combined (n = 43) for comparison to grade 3 tumours (n = 35), there was a significant association between grade and CE (Fisher's one sided exact test p = 0.008). The proportion of grade 1 and 2 tumours combined for NCE UC patients was 40/64 (63% (CI 51% to 74%) in comparison to 3/14 (21% (CI 0% to 42%)) for CE UC patients; none of the UC patients with a history of CE had Grade 1 tumours (Table 3).

### Age comparisons across patients

CE UC patients were significantly younger (median = 64 years (IQR 59 to 71)) than NCE UC patients (median = 71 years (IQR 65 to 76)) (ANOVA; p < 0.001) (t-test; p = 0.012). Further, there was no statistically significant difference in age when CE CTL and NCE CTL patients were compared; both groups had an average age of 54 years (Table 3).

### The relationship between chemical exposure and smoking across controls and urothelial cancer patients

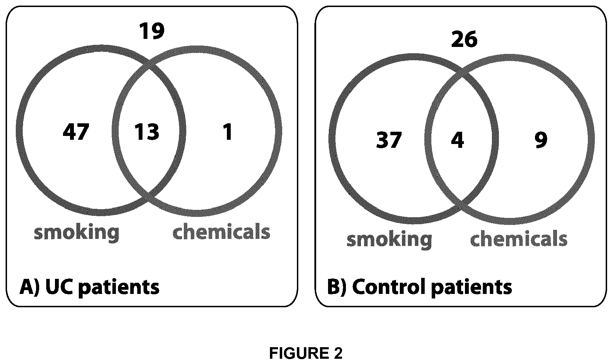

The proportion of patients with CE who also smoked (13/14 (93%)) was significantly higher in the UC subpopulation (hypergeometric p-value (greater) p₂ = 0.080; odds ratio = 5.18) in comparison to the CTL subpopulation (4/13 (31%)) (hypergeometric p-value (less) p₁ = 0.062; odds ratio = 0.32) (p = 0.025 (Zelen's test)). The odds ratios were not significant when the joint effect of smoking and CE was assessed independently for CTL and UC using a Fisher's exact test (Figure 2).

### Biomarkers with differential expression across patients with and without a history of chemical exposure

Four urinary biomarkers, i.e. CRP, IL-4, thrombomodulin, and IL-2 together with plasma biomarkers, MCP-1 and MMP9NGAL were pre-selected using Lasso. Urine IL-2, and the two plasma biomarkers, MCP-1 and MMP9NGAL, were less stable (bootstrap ≤0.11) than the three urinary biomarkers thrombomodulin, CRP and IL-4 (bootstrap ≥0.34) (Table 4).

Urinary thrombomodulin was the only biomarker that was differentially expressed across the NCE and CE subpopulations (Welch's t-test). Further, thrombomodulin was significantly higher in CE patients across both subpopulations, i.e. UC (mean NCE = 4.5 ng/ml; mean CE = 7.1 ng/ml) (one-sided t-test; p = 0.014) versus CTL (mean NCE = 4.5 ng/ml; mean CE = 6.2 ng/ml) (one-sided t-test; p = 0.043) groups demonstrating that the differential expression was independent of UC.

**Table 4 Differential analysis of pre-selected protein biomarker levels in patients with no history of chemical exposure and in patients with a history of chemical exposure**

| | **boot** | **NCE mean** | **NCE median** | **NCE IQR** | **CE mean** | **CE median** | **CE IQR** | **p-value** | **FDR** |
|---|---|---|---|---|---|---|---|---|---|
| uTM (ng/ml) | 0.51 | 4.5 | 4.0 | 2.6-5.5 | 6.78 | 5.9 | 4.2-8.1 | 0.002 | 0.013 |
| uCRP (ng/ml) | 0.53 | 1.0 | 0.9 | 0.7 - 1.2 | 1.3 | 1.1 | 0.7-1.4 | 0.041 | 0.123 |
| uIL-4 (pg/ml) | 0.34 | 6.7 | 6.6 | 6.6 - 6.6 | 7.0 | 6.6 | 6.6-6.7 | 0.088 | 0.176 |
| uIL-2 (pg/ml) | 0.11 | 5.9 | 5.6 | 5.2 - 6.4 | 6.9 | 5.8 | 5.3-6.4 | 0.118 | 0.176 |
| pMCP-1 (pg/ml) | 0.08 | 230.5 | 219.0 | 171.0-287.0 | 203.5 | 200.5 | 158.5-256.2 | 0.873 | 0.991 |
| pMMP9NGAL (U/ml) | 0.04 | 0.1 | 0.1 | 0.1-0.1 | 0.1 | 0.1 | 0.1-0.1 | 0.991 | 0.991 |

The table shows nominal and FDR adjusted p-values (Welch's t-test). Biomarker values were log₁₀ transformed prior to analyses. The bootstrap value (fraction) shown in column "boot" indicates how often the biomarker was selected by the Lasso procedure from 100 bootstrap datasets. TM thrombomodulin, CRP, C-reactive protein; IL-4, interleukin-4; IL-2, interleukin 2; MCP-1, monocyte chemoattractant protein 1; MMP9NGAL, matrix metalloproteinase neutrophil gelatinase associated lipocalin complex; FDR, false detection rate.

### Differential expression of thrombomodulin across subpopulations

Using a one-sided t-test and considering multiple testing using FDR, we observed that thrombomodulin levels in urine were significantly higher in CE patients, in the presence of dipstick protein and when urinary pH levels ≤6 (p = 0.003), but not in the presence of dipstick blood (p = 0.115).

Thrombomodulin and creatinine levels measured in urine were significantly correlated (Pearson correlation; r = 0.72, p < 0.001). For the remaining biomarkers r = < 0.5 (Pearson correlation).

### References

1. Dietrich H, Dietrich B. Ludwig Rehn (1849-1930)--pioneering findings on the aetiology of bladder tumours. World J Urol. 2001 Apr; 19(2):151-3. PubMed PMID: 11374319.
2. Jemal A, Bray F, Center MM, Ferlay J, Ward E, Forman D: "Global cancer Statistics" CA Cancer J Clin 2011, 61(2):69-90.
3. Mostafid H, Persad R, Kockelbergh R, Fawcett D: "Is it time to re-design the haematuria clinic?" BJU Int 2010, 105(5):585-588.
4. Michaud DS. Chronic inflammation and bladder cancer. Urol Oncol. 2007, 25: 260-268.
5. Michaud DS, Spiegelman D, Clinton SK, Rimm EB, Curhan GC, et al. (1999) Fluid intake and the risk of bladder cancer in men. N Engl J Med 340: 1390-1397.
6. Abogunrin F, O'Kane HF, Ruddock MW, Stevenson M, Reid CN, O'Sullivan JM, Anderson NH, O'Rourke D, Duggan B, Lamont JV, Boyd RE, Hamilton P, Nambirajan T, Williamson KE: "The impact of biomarkers in multivariate algorithms for bladder cancer diagnosis in patients with hematuria" Cancer 2011, 118(10):2641-50.
7. Guha N, Steenland NK, Merletti F, Altieri A, Cogliano V, Straif K. Bladder cancer risk in painters: a meta-analysis. Occup Environ Med. 2010 Aug; 67(8):568-73.
8. Smith EM, Miller ER, Woolson RF, Brown CK. Bladder cancer risk among auto and truck mechanics and chemically related occupations. Am J Public Health. 1985 Aug; 75(8):881-3.
9. Richardson K, Band PR, Astrakianakis G, Le ND. Male bladder cancer risk and occupational exposure according to a job-exposure matrix-a case-control study in British Columbia, Canada. Scand J Work Environ Health. 2007 Dec; 33(6):454-64.15.
10. Noon AP, Pickvance SM, Catto JW. Occupational exposure to crack detection dye penetrants and the potential for bladder cancer. Occup Environ Med. 2012 Apr; 69(4):300-1.
11. Kogevinas M, 't Mannetje A, Cordier S, Ranft U, Gonzalez CA, Vineis P, Chang-Claude J, Lynge E, Wahrendorf J, Tzonou A, Jöckel KH, Serra C, Porru S, Hours M, Greiser E, Boffetta P. Occupation and bladder cancer among men in Western Europe. Cancer Causes Control. 2003 Dec; 14(10):907-14.
12. Gaertner RR, Thériault GP. Risk of bladder cancer in foundry workers: a meta-analysis. Occup Environ Med. 2002 Oct; 59(10):655-63.16.
13. Harling M, Schablon A, Schedlbauer G, Dulon M, Nienhaus A. Bladder cancer among hairdressers: a meta-analysis. Occup Environ Med. 2010 May; 67(5):351-8.
14. Zelen M. The analysis of several 2 x 2 contingency tables, Biometrika, 58, pp. 129-137.
15. Hollander M. Nonparametric statistical methods. 2nd edn. 1999, New York; Chichester: John Wiley & Sons
16. Tibshirani R. Regression shrinkage and selection via the lasso. Journal of the Royal Statistical Society. Series B (Methodological), 1996; pp. 267-288.
17. Friedman J, Hastie T and Tibshirani R. Regularization paths for generalized linear models via coordinate descent. Journal of Statistical Software 2010; 33(1); 1 - 22.
18. Loghin A, Chibelean C, Orsolya M, Nechifor-Boil 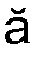 A, Nechifor-Boil 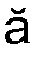 A, Borda A. Micropapillary urothelial carcinoma: an aggressive variant of urothelial carcinoma. Rom J Morphol Embryol. 2014;55(3):939-45.

## Claims

1. A method for detecting a history of exposure to a chemical(s) comprising measuring the concentration of thrombomodulin in a sample isolated from a subject, and determining whether the concentration of thrombomodulin is increased compared to control reference levels, wherein the subject has had or is suspected of having prior history of exposure to a chemical selected from the list comprising 1,1-dichloroethane, 2-mercaptobenzothiazole, β-naphthylamine or 2- naphthylamine, 4,4'-Methylenebis(2-chloroaniline) MOCA, 4-aminobiphenyl, aromatic amines, arsenic, auramine, benzidine, xenylamine, benzidine-derived dyes, aniline and azo dyes, benzo(a)pyrene, chlordimeform/4-COT, direct black 38, direct blue 6, direct brown 95, nitrobiphenyl, o-toluidine, polycyclic aromatic hydrocarbons, carbaryl, chlornaphazine, chlorophenols, creosotes, methylenedianiline, propoxur, trihalomethanes, antimony, asbestos, bifenthrin, cacodylic acid, carbamates, chromium, cadmium, nickel, leather dust, dichloropropene, nitrosamines, o-phenyl- phenol, organochlorine pesticides, p-cresidine, pesticides, pyrethins/pyrethroids, saccharin, tetrachloroethylene, phenyl β-naphthylamine, solvent red 164 as dye penetrant, solvent red 19 as dye penetrant or anti-rust agent, dinitrotoluenes, diazo printing ink, anti-neoplastic drugs, diabetic medications, and pain killers.

2. The method of claim 1, wherein the sample is a baseline sample from the subject.

3. The method of claim 1 or claim 2, wherein the sample is urine and/or serum and/or plasma.

4. The method of any preceding claim, wherein the subject suffers or has suffered from hematuria.

5. A method for detecting an increased risk of cancer in a subject suspected of a history of exposure to a chemical(s), comprising measuring the concentration of thrombomodulin in a sample isolated from the subject, and determining whether the concentration of thrombomodulin is increased compared to a control, wherein the subject has had or is suspected of having prior exposure to a chemical(s) selected from the list comprising 1,1-dichloroethane, 2-mercaptobenzothiazole, β-naphthylamine or 2-naphthylamine, 4,4'-Methylenebis(2-chloroaniline) MOCA, 4-aminobiphenyl, aromatic amines, arsenic, auramine, benzidine, xenylamine, benzidine-derived dyes, aniline and azo dyes, benzo(a)pyrene, chlordimeform/4-COT, direct black 38, direct blue 6, direct brown 95, nitrobiphenyl, o-toluidine, polycyclic aromatic hydrocarbons, carbaryl, chlornaphazine, chlorophenols, creosotes, methylenedianiline, propoxur, trihalomethanes, antimony, asbestos, bifenthrin, cacodylic acid, carbamates, chromium, cadmium, nickel, leather dust, dichloropropene, nitrosamines, o-phenyl-phenol, organochlorine pesticides, p-cresidine, pesticides, pyrethins/pyrethroids, saccharin, tetrachloroethylene, phenyl β- naphthylamine, solvent red 164 as dye penetrant, solvent red 19 as dye penetrant or anti-rust agent, dinitrotoluenes, diazo printing ink, anti-neoplastic drugs, diabetic medications, and pain killers,
wherein the cancer is bladder cancer.

6. The method of claim 5, wherein the bladder cancer is urothelial carcinoma.

7. The method of claim 5 or claim 6, wherein the increased risk of cancer is an increased risk of having a more aggressive tumour, preferably
wherein the tumor is a grade 3 tumor, or
the tumor is a stage 3 tumor or a stage 4 tumor.

8. The method of either one of claims 5 to 7, wherein the subject is less than 70 years old, preferably
wherein the subject is less than 65 years old, preferably
wherein the subject is less than 60 years old, preferably
wherein the subject is less than 55 years old, preferably
wherein the subject is less than 50 years old, preferably
wherein the subject is less than 45 years old.

9. The method of any one of claims 5 to 8, wherein the sample is urine, and/or preferably
wherein the subject suffers or has suffered from hematuria.

10. The method of any preceding claim, wherein the control is a reference concentration of thrombomodulin for detecting a history of exposure to a chemical(s) or detecting an increased risk of bladder cancer or of having a higher grade or higher stage tumour.

11. The method of any preceding claim, wherein the concentration of thrombomodulin is measured by binding of an antibody that binds specifically to thrombomodulin, preferably
wherein the concentration of thrombomodulin, is measured using biochip array technology, preferably
wherein the probe binds specifically to thrombomodulin, preferably
wherein the probe is an antibody that binds specifically to thrombomodulin.

## Patentansprüche

1. Verfahren zum Erfassen einer Expositiongeschichte gegenüber (einer) Chemikalie(n) zum Messen der Thrombomodulin-Konzentration in einer isolierten Probe von einem Probanden und zum Bestimmen, ob die Thrombomodulin-Konzentration im Vergleich zu Kontrollreferenzwerten erhöht ist, wobei bekannt ist oder vermutet wird, dass der Proband eine Vorgeschichte von Exposition gegenüber einer Chemikalie aufweist, ausgewählt aus der Liste, umfassend 1,1-Dichlorethan, 2-Mercaptobenzothiazol, β-Naphthylamin oder 2-Naphthylamin, 4,4'-Methylenbis(2-Chloranilin) MOCA, 4-Aminobiphenyl, aromatischen Aminen, Arsen, Auramin, Benzidin, Xenylamin, von Benzidin abgeleiteten Farbstoffen, Anilin- und Azofarbstoffe, Benzo(a)pyren, Chlordimeform/4-COT, Direktschwarz 38, Direktblau 6, Direktbraun 95, Nitrobiphenyl, o-Toluidin, polycyclischen aromatischen Kohlenwasserstoffen, Carbaryl, Chlornaphazin, Chlorphenole, Kreosote, Methylendianilin, Propoxur, Trihalomethane, Antimon, Asbest, Bifenthrin, Cacodylsäure, Carbamaten, Chrom, Cadmium, Nickel, Lederstaub, Dichloropropen, Nitrosamine, o-Phenylphenol, Organochlor-Pestiziden, p-Cresidin, Pestiziden, Pyrethinen/Pyrethroiden, Saccharin, Tetrachlorethylen, Phenyl-β-Naphthylamin, Lösungsmittelrot 164 als Farbstoffpenetrationsmittel, Lösungsmittelrot 19 als Farbstoffpenetrationsmittel oder Rostschutzmittel, Dinitrotoluolenen, Diazodruckfarbe, antineoplastischen Arzneistoffen, Medikamenten gegen Diabetes und Schmerzmitteln.

2. Verfahren nach Anspruch 1, wobei die Probe eine Probe aus der Grundlinie des Probanden ist.

3. Verfahren nach Anspruch 1 oder Anspruch 2, wobei die Probe Urin und/oder Serum und/oder Plasma ist.

4. Verfahren nach einem der vorhergehenden Ansprüche, wobei der Proband an Hämaturie leidet oder gelitten hat.

5. Verfahren zum Erfassen eines erhöhten Krebsrisikos bei einem Probanden, bei dem der Verdacht auf Exposition gegenüber einer Chemikalie(n) in der Vorgeschichte besteht, umfassend das Messen der Thrombomodulinkonzentration in einer aus dem Probanden isolierten Probe und das Bestimmen, ob die Thrombomodulinkonzentration im Vergleich zu einer Kontrolle erhöht ist, wobei bekannt ist oder vermutet wird, dass der Proband eine Vorgeschichte von Exposition gegenüber einer Chemikalie(n) aufweist, ausgewählt aus der Liste, umfassend 1,1-Dichlorethan, 2-Mercaptobenzothiazol, [3-Naphthylamin oder 2-Naphthylamin enthält, 4, 4'-Methylenbis (2-chloranilin) MOCA, 4-Aminobiphenyl, aromatischen Aminen, Arsen, Auramin, Benzidin, Xenylamin, von Benzidin abgeleiteten Farbstoffen, Anilin- und Azofarbstoffen, Benzo(a)pyren, Chlordimeform/4-COT, Direktschwarz 38, Direktblau 6, Direktbraun 95, Nitrobiphenyl, o-Toluidin, polycyclischen aromatischen Kohlenwasserstoffen, Carbaryl, Chlornaphazin, Chlorphenolen, Kreosoten, Methylendianilin, Propoxur, Trihalomethanen, Antimon, Asbest, Bifenthrin, Cacodylsäuren, Carbamaten, Chrom, Cadmium, Nickel, Lederstaub, Dichlorpropen, Nitrosaminen, o-Phenylphenol, Organochlorpestizide, p-Cresidin, Pestizide, Pyrethine / Pyreth-Überfälle, Saccharin, Tetrachlorethylen, Phenyl-β-Naphthylamin, Lösungsmittelrot 164 als Farbstoffpenetriermittel, Lösungsmittelrot 19 als Farbstoffpenetrations- oder Rostschutzmittel Dinitrotoluole, Diazodruckfarbe, antineoplastischen Arzneimitteln, Medikamenten gegen Diabetes und Schmerzmitteln, wobei der Krebs Blasenkrebs ist.

6. Verfahren nach Anspruch 5, wobei der Blasenkrebs ein Urothelkarzinom ist.

7. Verfahren nach Anspruch 5 oder Anspruch 6, wobei das erhöhte Risiko einer Krebserkrankung ein erhöhtes Risiko ist, an einem aggressiveren Tumor zu leiden, wobei vorzugsweise der Tumor ein Tumor der dritten Stufe oder ein Tumor im dritten Stadium oder im vierten Stadium ist.

8. Verfahren nach einem der Ansprüche 5 bis 7, wobei der Proband weniger als 70 Jahre alt ist, wobei vorzugsweise der Proband jünger als 65 ist, wobei vorzugsweise der Proband jünger als 60 ist, wobei vorzugsweise der Proband jünge als 55 ist, vorzugsweise der Proband jünger als 50 ist, vorzugsweise der Proband jünger als 45 ist.

9. Verfahren nach einem der Ansprüche 5 bis 8, wobei die Probe Urin ist, und/oder vorzugsweise wobei der Proband an Hämaturie leidet oder gelitten hat.

10. Verfahren nach einem der vorhergehenden Ansprüche, wobei die Kontrolle eine Referenzthrombomodulin-Konzentration zur Erfassung einer Expositionsgeschichte gegenüber einer Chemikalie(n) oder zum Erfassen eines erhöhten Risikos an einer Blasenkrebserkrankung zu leiden oder einen Tumor mit einer höheren Stufe oder einem höheren Stadium aufzuweisen.

11. Verfahren nach einem der vorhergehenden Ansprüchen, wobei die Thrombomodulin-Konzentration durch Bindung eines Antikörpers gemessen wird, dass spezifisch an Thrombomodulin bindet, wobei bevorzugt die Thrombomodulin-Konzentration unter Verwendung von Biochip-Array-Technologie gemessen wird, wobei die Sonde spezifisch vorzugsweise an Thrombomodulin bindet, wobei die Sonde vorzugsweise ein Antikörper ist, der spezifisch an Thrombomodulin bindet.

## Revendications

1. Procédé de détection d'un antécédent d'exposition à un ou plusieurs produits chimiques comprenant la mesure de la concentration de thrombomoduline dans un échantillon isolé d'un sujet et la détermination du fait que la concentration de thrombomoduline est augmentée par rapport à des niveaux de référence de contrôle, le sujet ayant eu ou étant soupçonné d'avoir un antécédent d'exposition à un produit chimique choisi dans la liste comprenant le 1,1-dichloroéthane, le 2-mercaptobenzothiazole, la β-naphthylamine ou la 2-naphthylamine, la 4,4'-méthylènebis(2-chloroaniline) MOCA, le 4 - aminobiphényle, les amines aromatiques, l'arsenic, l'auramine, la benzidine, la xénylamine, les colorants dérivés de la benzidine, les colorants aniline et azoïques, le benzo(a)pyrène, le chlordiméform/4-COT, le noir direct 38, le bleu direct 6, le brun direct 95, le nitrobiphényle, l'o-toluidine, les hydrocarbures aromatiques polycycliques, le carbaryl, la chlornaphazine, les chlorophénols, les créosotes, la méthylènedianiline, le propoxur, les trihalométhanes, l'antimoine, l'amiante, la bifenthrine, l'acide cacodylique, les carbamates, le chrome, le cadmium, le nickel, la poussière de cuir, le dichloropropène, les nitrosamines, l'o-phénylphénol, les pesticides organochlorés, la p-crésidine, les pesticides, les pyréthines/pyréthroïdes, la saccharine, le tétrachloroéthylène, le phényl β-naphtylamine, le solvant rouge 164 comme pénétrant coloré, le solvant rouge 19 comme pénétrant coloré ou agent antirouille, les dinitrotoluènes, l'encre d'imprimerie diazoïque, les médicaments antinéoplasiques, les médicaments contre le diabète, et les analgésiques.

2. Procédé selon la revendication 1, dans lequel l'échantillon est un échantillon de base du sujet.

3. Procédé selon la revendication 1 ou la revendication 2, dans lequel l'échantillon est de l'urine et/ou du sérum et/ou du plasma.

4. Procédé selon l'une quelconque des revendications précédentes, dans lequel le sujet souffre ou a souffert d'hématurie.

5. Procédé de détection d'un risque accru de cancer chez un sujet soupçonné d'un antécédent d'exposition à un ou plusieurs produits chimiques, comprenant la mesure de la concentration de thrombomoduline dans un échantillon isolé du sujet et la détermination du fait que la concentration de thrombomoduline est augmentée par rapport à un contrôle, le sujet ayant eu ou étant soupçonné d'avoir un antécédent d'exposition à un ou plusieurs produits chimiques choisis dans la liste comprenant le 1,1-dichloroéthane, le 2-mercaptobenzothiazole, la [β-naphthylamine ou la 2-naphthylamine, la 4,4'-méthylènebis (2-chloroaniline) MOCA, le 4-aminobiphényle, les amines aromatiques, l'arsenic, l'auramine, la benzidine, la xénylamine, les colorants dérivés de la benzidine, les colorants aniline et azoïques, le benzo(a)pyrène, le chlordiméform/4-COT, le noir direct 38, le bleu direct 6, le brun direct 95, le nitrobiphényle, l'o-toluidine, les hydrocarbures aromatiques polycycliques, le carbaryl, la chlornaphazine, les chlorophénols, les créosotes, la méthylènedianiline, le propoxur, les trihalométhanes, l'antimoine, l'amiante, la bifenthrine, l'acide cacodylique, les carbamates, le chrome, le cadmium, le nickel, la poussière de cuir, le dichloropropène, les nitrosamines, l'o-phénylphénol, les pesticides organochlorés, la p-crésidine, les pesticides, les pyréthines/pyréthroïdes, la saccharine, le tétrachloroéthylène, le phényl β-naphthylamine, le solvant rouge 164 comme pénétrant coloré, le solvant rouge 19 comme pénétrant coloré ou agent antirouille, les dinitrotoluènes, l'encre d'impression diazoïque, les médicaments antinéoplasiques, les médicaments contre le diabète, et les analgésiques,
dans lequel le cancer est un cancer de la vessie.

6. Procédé selon la revendication 5, dans lequel le cancer de la vessie est un carcinome urothélial.

7. Procédé selon la revendication 5 ou la revendication 6, dans lequel le risque accru de cancer est un risque accru d'avoir une tumeur plus agressive, de préférence
dans lequel la tumeur est une tumeur de grade 3, ou
la tumeur est une tumeur de stade 3 ou une tumeur de stade 4.

8. Procédé selon l'une quelconque des revendications 5 à 7, dans lequel le sujet a moins de 70 ans, de préférence
dans lequel le sujet a moins de 65 ans, de préférence
dans lequel le sujet a moins de 60 ans, de préférence
dans lequel le sujet a moins de 55 ans, de préférence
dans lequel le sujet a moins de 50 ans, de préférence
dans lequel le sujet a moins de 45 ans.

9. Procédé selon l'une quelconque des revendications 5 à 8, dans lequel l'échantillon est de l'urine, et/ou de préférence
dans lequel le sujet souffre ou a souffert d'hématurie.

10. Procédé selon l'une quelconque des revendications précédentes, dans lequel le contrôle est une concentration de référence de thrombomoduline pour détecter un antécédent d'exposition à un ou plusieurs produits chimiques ou détecter un risque accru de cancer de la vessie ou d'avoir une tumeur de grade supérieur ou de stade supérieur.

11. Procédé selon l'une quelconque des revendications précédentes, dans lequel la concentration de thrombomoduline est mesurée par liaison d'un anticorps qui se lie spécifiquement à la thrombomoduline, de préférence
dans lequel la concentration de thrombomoduline est mesurée à l'aide de la technologie de réseau de biopuces, de préférence
dans lequel la sonde se lie spécifiquement à la thrombomoduline, de préférence
dans lequel la sonde est un anticorps qui se lie spécifiquement à la thrombomoduline.
